# EUROPÄISCHE PATENTSCHRIFT

(11) **EP 1 589 944 B1**
(45) Veröffentlichungstag und Bekanntmachung des Hinweises auf die Patenterteilung: **23.11.2011**
(21) Anmeldenummer: 04708685.5
(22) Anmeldetag: 06.02.2004
(51) Int. Cl.: A61K 9/00

(54) **PHARMAZEUTISCHE ZUBEREITUNGEN UND VERFAHREN ZU DEREN HERSTELLUNG**
PHARMACEUTICAL PREPARATIONS AND A METHOD FOR THEIR PRODUCTION
PREPARATIONS PHARMACEUTIQUES ET LEUR PROCEDE DE PRODUCTION

(30) Priorität: 06.02.2003 AT 1882003
(43) Veröffentlichungstag der Anmeldung: 02.11.2005
(73) Patentinhaber: Gebro Pharma GmbH, 6391 Fieberbrunn (AT)
(72) Erfinder: AMAN, Wolfgang, A-6300 Wörgl (AT); CAPELLO, Heinz, A-6380 St. Johann (AT)
(74) Vertreter: Wildhack & Jellinek
(86) Internationale Anmeldenummer: PCT/AT2004/000039
(87) Internationale Veröffentlichungsnummer: WO 2004/069132

(56) Entgegenhaltungen:
- EP-A- 0 350 701
- EP-A- 0 390 369
- WO-A-00/59467
- WO-A-94/10994
- US-A- 5 904 937

## Beschreibung

Die Erfindung bezieht sich auf die Geschmacksmaskierung von pharmazeutischen Zubereitungen, welche als Wirkstoff mindestens ein Propionsäure-Derivat bzw. Profen enthalten, zur peroralen Applikation.

Insbesondere bezieht sich die Erfindung auf neue pharmazeutische Zubereitungen, wie Pulver, Granulate und Tabletten, die unmittelbar vor der Anwendung durch die Einbringung in bzw. Zugabe von Wasser in eine trinkbare Form übergeführt werden. Die auf diese Weise hergestellten trinkbaren Zubereitungen sind hilfreich bei der Behandlung von Schmerzen, Entzündungen und Fieber. Obwohl die Vorteile trinkbarer Zubereitungen allen Arten von Patienten zu Gute kommen, sind sie speziell geeignet für Kinder, für ältere Patienten und für Patienten mit Schluckbeschwerden.

Die Erfindung betrifft weiters die Herstellung der neuen Zubereitungen, die schon angesprochenen trinkbaren Zubereitungen und deren Herstellung sowie die Verwendung der Wirkstoffe in einer spezifischen Form.

### HINTERGRUND DER ERFINDUNG

Die Propionsäure-Derivate oder genauer gesagt, Arylpropionsäure-Derivate stellen eine Gruppe von Wirkstoffen mit hoher Entzündungshemmungs-Wirkung dar. Zum Teil werden diese Wirkstoffe auch als "Profene" bezeichnet. Zu deren wichtigsten Vertretern gehören Ibuprofen, Flurbiprofen, Ketoprofen, Naproxen, Fenprofen, Carprofen, die sowohl als Racemat, als auch enantiomer-rein vorliegen können, wie z.B. Dexibuprofen, sowie deren pharmazeutisch akzeptablen Salze, Ester u.dgl.

Einer der wichtigsten Wirkstoffe dieser Gruppe ist das Dexibuprofen [S(+)-Ibuprofen]; es ist das rechts drehende Enantiomer des Ibuprofens. Das rechts drehende Enantiomer ist für die antiphlogistische, antipyretische und analgetische Wirkung verantwortlich. Aufgrund des schlechten Geschmacks vieler Propionsäure-Derivate und insbesondere des Dexibuprofens und auch des racemischen Ibuprofens werden dieselben in der Regel in Form von überzogenen Tabletten verabreicht bzw. eingenommen. Wünschenswert sind neben als solche zu verabreichende Tabletten jedoch trinkbare Formulierungen, vor allem für ältere Patienten und/oder Kinder, welche Trinklösungen bzw. -suspensionen vorziehen. Dies gilt insbesondere auch für Personen, welche unter Schluckbeschwerden leiden.

Das wesentliche Problem der Formulierung trinkbarer Zubereitungen mit Propionsäure-Derivaten, wie insbesondere Dexibuprofen und Ibuprofen, ist der ausgesprochen schlechte und unangenehme Geschmack dieser Substanzen. So weist Ibuprofen einen sehr bitteren Geschmack auf. Dexibuprofen, der tatsächlich wirksame Bestandteil von Ibuprofen, ist nicht nur in seiner therapeutischen Wirkung besonders potent, sondern bedauerlicherweise auch in der Intensität seines unangenehmen Geschmacks. Bei Einnahme des Wirkstoffes alleine oder in einer Arzneiform ohne Geschmacksmaskierung löst die Substanz im Mund- und Rachenbereich bis hinein in die Speiseröhre ein starkes Brennen aus, das bisher eine ungeschützte Einnahme unmöglich gemacht hat. Diese Geschmacksirritation ist umso stärker, je niedriger-viskos die mit diesem Wirkstoff bereiteten trinkbaren Zubereitungen sind, da die üblicherweise verwendeten viskositätserhöhenden Substanzen zumeist auch geschmacksneutralisierend wirken.

Interessanterweise wird in der EP 0 753 296 A2 für Ibuprofen eine Maskierung des bitteren Substanzgeschmackes durch die alleinige Verwendung von S(+)-Ibuprofen beschrieben. S(+)-Ibuprofen schmeckt zwar nicht so bitter wie das racemische Ibuprofen, hinterlässt aber den oben beschriebenen brennenden Nachgeschmack, der als noch unangenehmer empfunden wird.

Die meisten Erfindungen des Standes der Technik beziehen sich jedoch auf die Geschmacksmaskierung des Ibuprofen-Racemates. Allgemeiner Stand der Technik zur Geschmacksmaskierung unangenehm schmeckender Arzneistoffe ist das Überziehen der Substanzpartikel. Für diese Art der Maskierung des Geschmacks von Ibuprofen werden verschiedene Polymere beschrieben, z.B. Ethylcellulose (WO 00/727368), Hydroxypropymethylcellulose-phthalat (EP 0 366 101 A2), oder Eudragit L30D (WO 91/15194).

*Auch bei den in der* EP 350701 A1 *beschriebenen pharmazeutischen Zubereitungen für die Bereitung von Trinkzubereitungen von Wirkstoffen mit analgetischer und entzündungshemmender Wirkung ist vorgesehen, dass diese in fein zerteilter Form vorliegen, wobei die feinen Wirkstoff-Teilchen zwingend mit Substanzen, welche den unangenehmen Geschmack des Wirkstoffes maskieren, beschichtet sind.*

Neben den klassischen Überzugsmaterialien wird *weiters* auch die Möglichkeit der Einbettung von Ibuprofen in pharmazeutischen Fetten erwähnt. Dies soll die Herstellung geschmacksneutraler Kautabletten ermöglichen (WO 92/21328).

Das Prinzip ist bei *allen* Maskierungs-Methoden gemäß den bisher genannten Druckschriften ähnlich: Der schlecht schmeckende Wirkstoff wird mit einem neutral schmeckenden Hilfsstoff umhüllt. Diese Umhüllung ist jedoch nur durch ein aufwendiges und kostenintensives Verfahren möglich.

Eine andere Methode der Geschmacksmaskierung für eine trinkbare Ibuprofen-Zubereitung ist in der EP 0 418 043 A1 beschrieben. Gemäß dieser Schrift wird eine Geschmacksmaskierung von wasserlöslichen Ibuprofen-Salzen (Natriumibuprofenat) durch die Zugabe von Natriumbicarbonat, Natriummonohydrogenphosphat oder Natriumtricitrat zur Lösung erreicht. Auch Arginin wird als alkalisierende Komponente beschrieben, um Ibuprofen in Lösung zu bringen und Trinklösungen herzustellen (WO 95/00134). Diese Art der Geschmacksmaskierung ist für Dexibuprofen allerdings nicht anwendbar. Zwar wird der brennende Geschmack des "sauren" Dexibuprofens durch Neutralisierung, u.a. mit den genannten Substanzen, reduziert, an seine Stelle tritt jedoch der intensiv laugige Geschmack des gelösten Dexibuprofen-Salzes. Dieser Seifengeschmack ist ebenfalls äußerst unangenehm und kann nicht abgedeckt werden.

Die WO 00/59467 beschreibt eine trinkbare Suspension mit Ibuprofen, die durch die Anwesenheit eines viskositätserhöhehden Hilfsstoffes, einer geladenen Substanz und eines Konservierungsmittels charakterisiert ist. Viskositätserhöhende Stoffe verlangsamen jedoch die Dispersion einer festen Zubereitung in Wasser. Zudem betragen die Volumina der hoch viskosen Zubereitungen bloß ca. 5 - 20 ml pro Applikation (Einzeldosis). Größere Volumina, also etwa 150 - 250 ml, werden vom Patienten nur in Form von niedrig viskosen Lösungen akzeptiert.

Die EP 0 945 132 A2 weist auf die Reduktion des brennenden Nachgeschmackes von Ibuprofen durch die Zugabe von Fumarsäure hin. Entgegen den Behauptungen der Erfinder in dieser Schrift ist Fumarsäure jedoch toxikologisch nicht unbedenklich. Zudem ist sie zur Unterdrückung der Geschmacksirritation durch Dexibuprofen nicht geeignet.

Zum Stand der Technik auf dem Gebiet der Geschmacks-Maskierung von Profene enthaltenden pharmazeutischen Zubereitungen seien hier als verschiedene Wege zur Erreichung dieses Zieles aufzeigende Druckschriften die WO 95/01321 A1, WO 94/10994 A1, WO 00/59467 A1, WO 99/17748 A1 sowie die EP 390369 A2 genannt, auf welche später noch ausführlich eingegangen werden wird.

*Ergänzend ist noch die* US 5904937 A1 *zu nennen:*

*Diese Schrift betrifft nicht eine zuerst in fester Form vorliegende, dann aber zwingend für eine Dispergierung in Wasser, zu einer Trink-Suspension vorgesehene Zubereitung, sondern eine Kautablette, in welcher der Profen-Wirkstoff mit einer Partikelgröße im Bereich von 100 bis 1000 µm vorliegt, wobei eine Geschmacksmaskierung gezielt durch Einarbeiten von mikrokristalliner Cellulose erreicht werden soll.*

*Diese Schrift enthält keine Aussagen über die Größe der Teilchen des Wirkstoffes selbst, sondern die dortigen Angaben der Teilchengröße bezieht sich auf die Partikel aus dem Gemisch von Wirkstoff-Teilchen plus mikrokristalliner Cellulose und den anderen Bestandteilen. In diesem Gemisch können die Wirkstoff-Partikel selbst eine Größe von z.B. bloß 1µm haben.*

*Dieser Schrift liegt nicht die essentielle Erkenntnis zugrunde, dass Wirkstoff-Partikel mit Teilchengrößen von unter 100 µm zumindest zu 95 % nicht vorliegen dürfen.*

### ZIEL DER ERFINDUNG

Ziel der Erfindung ist es, eine pharmazeutische Zubereitung in fester Form, also in Form von Tabletten, Granulaten, Pulvern od.dgl., mit mindestens einem Propionsäure-Derivat, insbesondere aus der Gruppe der Profene, zu entwickeln, welche, bevorzugterweise unmittelbar, vor der Anwendung durch Einbringen in oder Zugabe von Wasser in eine trinkbare Form, also in eine Trink-Zubereitung, übergeführt wird.

Diese trinkbare Zubereitung sollte wohlschmeckend sein und sollte keine Geschmacksirritation hinterlassen. Sie bzw. deren Ausgangs-Zubereitung sollte weiters einfach und kostengünstig hergestellt werden können. Die flüssige Zubereitung sollte eine niedrige Viskosität aufweisen, und der Wirkstoff soll frei, also nicht durch einen Film abgedeckt vorliegen, damit ein schnelles Dispergieren der in fester Form vorliegenden Zubereitung im Wasser ermöglicht wird und dieselbe dann vom Patienten auch problemlos und ohne Ekel angenommen wird:

### BESCHREIBUNG DER ERFINDUNG

Im Zuge eingehender Untersuchungen wurde überraschenderweise festgestellt, dass bei gezieltem Einsatz von ganz bestimmten ausgewählten Korngrößenfraktionen der in Rede stehenden Wirkstoffe, wie insbesondere Ibuprofen und Dexibuprofen, die oben beschriebenen, äußerst unangenehmen Geschmacksirritationen zumindest weitestgehend und in den meisten Fällen überhaupt vermieden werden können. Dieser Erkenntnis lag die Beobachtung zugrunde, dass jedenfalls nach Entfernung des Fein- bzw. Feinstanteils der Partikel der in Rede stehenden Wirkstoffe der bitter-brennende Geschmack wesentlich reduziert war oder überhaupt nicht auftrat.

Gegenstand der vorliegenden Erfindung ist somit eine neue pharmazeutische *Zubereitung in fester Pulver-, Granulat oder Tablettenform, welche mindestens ein extrem unangenehm bitteren bzw. brennenden Geschmack aufweisendes Propionsäure-Derivat bzw. Profen, vorzugsweise Dexibuprofen oder racemisches Ibuprofen, als Wirkstoff sowie weiters pharmazeutische Hilfs- und Zusatzstoffe enthält, für die Bereitung einer Trink-Zubereitung durch Dispergierung in Wasser, welche dadurch gekennzeichnet ist, dass der genannte unangenehm bittere bzw. brennende Geschmack der mit der Feststoff-Zubereitung hergestellten Trink-Zubereitung unterdrückt, maskiert bzw. vermieden ist, indem das in der Feststoff-Zubereitung jeweils enthaltene Propionsäure-Derivat in Form von - von Partikeln aller anderen Komgrößen-Fraktionen im Wesentlichen befreiten, zumindest zu 95 %, der Komgrößen-Fraktion von 100 bis 1000 µm angehörenden - Partikeln vorliegt, und die Propionsaure-Derivat-Partikel keinen Überzugsfilm bzw. keinen, deren Kontakt bzw. Benetzung mit Wasser hemmenden Überzugsfilm od. dgl. aufweisen.*

Im Sinne der Geschmacksmaskierung besonders bevorzugt sind wie soeben beschriebene pharmazeutische Zubereitungen mit jeweils noch engeren Korngrößen-Fraktionsbereichen des in ihnen enthaltenen Propionsäure-Derivats, wie sie dem Anspruch 2 zu entnehmen sind.

Der Grobanteil der Wirkstoff-Partikel ist nach oben hin insbesondere dadurch begrenzt, dass ansonsten die Homogenität des Wirkstoffes in den jeweiligen festen Zubereitungen bzw. in der trinkbaren Zubereitung nicht gewährleistet ist.

Gemäß der vorliegenden Erfindung müssen in den neuen Zubereitungen zumindest zu 90 % Propionsäure-Derivat-Partikel vorliegen, deren Größe innerhalb des im Anspruch 1 genannten Bereichs von *100* bis *1000* µm, liegt, wobei Reste und letztlich auch Spuren bitteren Geschmacks bei den im Anspruch 2 genannten, weiter eingeengten Partikelgrößen-Bereichen gänzlich eliminiert sind. Es kommt dieses strikte Herausgreifen eines breiteren und bevorzugterweise der noch schmäleren Partikelgrößen-Bereichsbänder durch die strikte Formulierung im Anspruch 1 deutlich zum Ausdruck, dass dort die innerhalb der genannten Partikelgrößen-Bandbreiten liegenden Wirkstoff-Teilchen zumindest zu 95 %, von Teilchen, welche eine Größe unterhalb der genannten unteren Bereichsgrenze und andererseits eine Größe oberhalb der oberen Grenze des genannten Bereichs aufweisen, befreit sein müssen.

Es sind also erfindungsgemäß - und dies ist für den angestrebten Effekt ausschlaggebend - praktisch scharfe Grenzen gezogen, welche die in Richtung Geschmacks-Maskierung tatsächlich effektive Teilchengrößen der geschmacks-irritierenden Wirkstoffe definieren.

Sowohl bei der Angabe der unteren als auch der oberen Grenze der Teilchengrößen-Bereiche beziehen sich die in den Ansprüchen 1 und 2 genannten Zahlen nicht auf Bereiche von "mittleren" Teilchengrößen, vielmehr ist ganz gezielt der zumindest 95 %ige Ausschluss von Wirkstoffpartikeln mit Größen unterhalb und von solchen oberhalb der gemäß den Ansprüchen 1 und 2 einzuhaltenden Bereichsgrenzen vorgesehen.

Die gesamte Erfindung ist darauf ausgerichtet, dass sie eben nicht auf einer Angabe etwa der Art, dass
"die Teilchen mittlere Teilchengrößen im Bereich zwischen x µm und y µm aufweisen",
beruht, was ein Vorliegen von Teilchengrößen unterhalb und oberhalb jeweiliger Bereichsgrenzen aufweisenden, also von kleineren bzw. größeren und eben auch von wesentlich kleineren bzw. größeren Partikeln keineswegs ausschließen würde.

Es bildet also gerade die in ihren Grenzen definierte Wirkstoff-Partikelgröße und deren gezielte Auswahl den Kern der vorliegenden Erfindung, und es spielt bei Betrachtung und Würdigung des Standes der Technik gerade dieser Aspekt eine entscheidende Rolle:

So ist zu dem in der WO 95/01321 A1 geoffenbarten Gegenstand festzuhalten, dass dort im Wesentlichen auch das gleiche Ziel verfolgt wird, wie erfindungsgemäß, also das Ausschalten des bitter-brennenden Geschmacks von Ibuprofen in dasselbe enthaltenden Zubereitungen. Um diesem Ziel nahe zu kommen, gehen die Autoren dieser WO-A1 einen gänzlich anderen und wesentlich aufwendigeren Weg, als dies erfindungsgemäß der Fall ist: Sie geben die Anweisung, eine "neomorphe" Ibuprofen-Zubereitung mit einen ganz bestimmte Grundgestalt aufweisenden Teilchen aus "amorphem Ibuprofen" zu verabreichen.

Dass die gesamte WO-A1 diesen Weg einer speziellen Gestaltung der amorphen Ibuprofen-Partikel zum Gegenstand hat, zeigen deren Ansprüche deutlich, von welchen kein einziger irgendeine Angabe bezüglich gezielt ausgewählter Partikelgrößen enthält.

Die vorliegende Erfindung geht auf Basis entsprechender Ergebnisse von Versuchsreihen und auf Grund von daraus gewonnenen Erkenntnisse einen gänzlich anderen, wesentlich einfacheren und reproduzierbaren Weg: Sie sorgt ganz gezielt dafür, dass Wirkstoffpartikel mit Größen unterhalb bzw. oberhalb der im jeweiligen Anspruch angegebenen Größen-Unter- bzw. Obergrenzen in der Zubereitung praktisch eben nicht oder aber höchstens in, wie gefunden wurde, nicht geschmacksbeeinflussend wirksamen Mengen vorliegen.

In dem Beschreibungsteil der oben erwähnten WO-A1 sind zwar Teilchengrößen und bevorzugte Teilchengrößen-Bereiche genannt, dies hat jedoch mit einer etwaigen Erkenntnis, die dortige Auswahl würde eine Geschmacksmaskierung bewirken können, nichts zu tun. Dementsprechend sind aus den Teilchengrößen-Angaben dort keine Konsequenzen in dieser Richtung gezogen worden.

Neben dem Faktum, dass wie gemäß der WO-A1 vorgesehenes amorphes Ibuprofen kein Dexibuprofen ist, muss darüber hinaus deutlich darauf verwiesen werden, dass sich die Teilchengrößen (-Bereichs)-Angaben in dieser Schrift alle auf "averaged particle sizes", also auf mittlere Teilchengrößen beziehen, was die obigen Ausführungen hinsichtlich des absoluten Nichterkennens der Bedeutung einer gezielten und sauber begrenzenden Partikelgrößenauswahl durch die Autoren der WO-A1 deutlich bestätigt. Zuletzt ist zur weiteren Bestätigung dieses Faktums auf die Ausführungen der Seite 11, Zeilen 9 bis 13 der WO-A1 hingewiesen, wo von "smaller averaged particle sizes" und weiters von Teilchen von "unter etwa 50 µm" die Rede ist.

Für die ebenfalls eine Geschmacksmaskierung zum Gegenstand habende WO 94/10994 A1 gilt, dass die dort geoffenbarte Erfindung ebenfalls keineswegs auf einer etwaigen Erkenntnis beruht, dass die Wirkstoff-Teilchengröße und deren strikte Festlegung auf einen bestimmten Bereich für eine tatsächliche Erreichung des Ziels entscheidend ist, und dass daher dort auch nicht die Nutzung einer solchen nicht-existenten Erkenntnis beschrieben sein kann.

Der Schwerpunkt des Gegenstandes dieser WO-A1 liegt in der Schaffung eines wirksamen Karbonat/Kohlendioxid-Entwicklungssystems für eine Brause-Zubereitung mit Ibuprofen als Wirkstoff, welche geschmacksmaskiert ist. Die in dieser Schrift genannten Teilchengrößen ermöglichen eine rasche Verteilung und Auflösung der Wirkstoff-Teilchen in der fertig bereiteten Brausezubereitung, wobei dort eine wesentliche Grundbedingung darin besteht, dass sie als "klare wässerige Lösung" vorliegen soll. Dies bedeutet, dass das Ibuprofen in der Zubereitung gar nicht als solches, also nicht mehr "frei" vorliegt, sondern infolge des dort vorgesehenen Überschusses an Natriumcarbonat im CO₂-Entwicklungs-System eben als Natriumsalz des Ibuprofens, also als Natrium-Ibuprofen, das selbstverständlich nicht mehr die Eigenschaften des freien Ibuprofens, also eben nicht mehr dessen bitter-brennenden Geschmack aufweist.

Beim Kontakt der in der WO 94/10994 A beschriebenen Zubereitung mit Wasser löst sich das "saure" Ibuprofen infolge des basischen Milieus, und, um dieses Lösen bei gleichzeitiger Umwandlung bzw. Überführung in das Natriumsalz zu erleichtern, wird dort ein für diesen Löse-Vorgang als günstig angesehener Bereich der Größe der Teilchen des eben nur im nicht gelösten Zustand in "freier" Form in der Zubereitung enthaltenden Ibuprofens vorgeschrieben.

Bezüglich der eklatanten Änderung der Eigenschaften von Ibuprofen als Folge der gemäß dieser WO-A1 beim Bereiten der Trinklösung unvermeidlich eintretenden Überführung in das Natriumsalz vor der Verabreichung soll insbesondere auf die Zeilen 9 bis 17 auf Seite 5 der WO-A1 hingewiesen werden.

Die WO 00/59467 A1 betrifft eine Ibuprofen-Trinksuspension, in welcher die lösliche Fraktion des Wirkstoffs weniger als 10 % des in der Suspension vorhandenen Ibuprofens betragen soll, bzw. die trockene Ausgangs-Zubereitung für die Trinksuspension. Es soll die Größe der Körner der in Form eines Granulates vorliegenden, jederzeit in die Suspension überführbaren, Feststoff-Form aufweisenden Zubereitung im Extremfall 50 bis 1000µm betragen, und die in dieser galenischen Applikationsform vorliegenden Kristallite des eigentlichen Wirkstoffs sollen gemäß der WO-A1 eine Größe von "unter 500µm" aufweisen.

Zu diesen Angaben ist massiv darauf zu verweisen, dass z.B. die in den 50µm großen Körnern der außer dem Ibuprofen zumindest noch Stärke, Zucker u. dgl. als Träger enthaltenden Zubereitung vorliegenden Wirkstoff-Kristallite wesentlich geringere Teilchengrößen aufweisen müssen, dass also dort die Teilchengröße des Wirkstoffes selbst weit unter 50µm liegen muss. Gerade von derartigen Kleinst-Partikeln sollen jedoch die gemäß der vorliegenden Erfindung in der pharmazeutischen Zubereitung eingesetzten Ibuprofen-Partikel "befreit" sein.

Es soll ergänzend darauf verwiesen werden, dass wie in Seite 3, Zeilen 1 bis 4 der WO-A1 geoffenbart, die Ein- bzw. Aufbringung des Wirkstoffes auf die Träger-Granulat-Teilchen z.B. durch Bepudern derselben mit dem Ibuprofen-Teilchen erfolgen kann, was bedeutet, dass dieselben eben tatsächlich wesentlich kleiner sein müssen als die Trägerstoff-Teilchen.

Es ist also auch in dieser WO-A1 keine Anregung und kein Hinweis auf einen gezielten Einsatz eines scharf begrenzten Partikelgrößen-Bereichs des Ibuprofens zur Erreichung der Geschmacksmaskierung gegeben.

Auch dem Gegenstand der EP 390369 A2 liegt weder die Erkenntnis noch wenigstens eine Vermutung zugrunde, dass allein durch gezielte Begrenzung des Wirkstoffpartikel-Größenbereiches, insbesondere nach unten, zu besonders geringen Partikelgrößen hin, der überraschende Effekt der Geschmacksmaskierung, wie er der vorliegenden Erfindung zu Grunde liegt, erzielbar sein könnte.

In dieser EP-A1 liegt ist der Schwerpunkt der dort sehr verdünnt vorliegenden Ibuprofen-Zubereitungen auf dem Einsatz bestimmter ausgewählter Puffer-Systeme.

Die dort enthaltene Empfehlung, nicht "zu kleine Ibuprofen-Partikel" einzusetzen, hat mit einer etwa beabsichtigten Geschmacksmaskierung nichts zu tun, sie zielt vielmehr daraufhin ab, dass es zu keinem Aufschwimmen der Teilchen - beispielsweise infolge der Wirkung der Oberflächenspannung des Wassers kommen soll. Vice versa soll die zubereitete Suspension keine zu großen Wirkstoffpartikel enthalten, um ein Absinken derselben zu verhindern.

Deutlich ist zu den in der EP-A1 genannten konkreten Zahlenangaben bezüglich der Partikelgrößen darauf zu verweisen, dass sich, siehe Seite 2, Zeilen 41, 42 der EP-A1, diese Angaben ebenfalls auf "mittlere" Teilchengrößen im Bereich von 30 bis 250µm beziehen.

Die WO 99/17748 A1 betrifft schließlich eine Methode der Maskierung von Arzneimitteln durch Nass-Granulierung mit mikrokristallinen Cellulose-Zubereitungen und nachheriger Sphäronisierung in kugelige Aggregate mit glatter Oberfläche und Teilchengrößen - was sich auf die Granulat-Partikel und nicht auf die Wirkstoff-Partikel bezieht - von 1 bis 1000µm, wobei insbesondere auf Seite 3, Zeilen 26, 27 dieser Schrift hinzuweisen ist, wo nur von "bis zu 1000 µm" ohne jegliche Nennung einer Untergrenze die Rede ist. Im Anspruch 1 dieser WO-A1 ist weiters von einer Granulat-Partikelgröße, also eben nicht von einer Wirkstoff-Partikelgröße von mindestens 100 µm die Rede, was selbstverständlich jegliche Teilchengrößen-Obergrenze offen lässt.

Auch in dieser WO-A1 fehlt jegliche Andeutung eines eventuellen Erkennens oder wenigstens Vermutens einer Kritizität des zu wählenden Partikelgrößen-Bereiches des Geschmacks-Irritationen hervorrufenden Wirkstoffes im Hinblick auf die an sich auf recht einfachem Weg erreichte Geschmacks-Maskierung, wie sie der vorliegenden Erfindung zugrunde liegt.

Gemäß allen soeben näher behandelten Druckschriften dienen die dort geoffenbarten Eingrenzungen der Partikelgrößen des aktiven Wirkstoffs - von welchen keine den erfindungsgemäß einzuhaltenden Kriterien voll entspricht - in keinem einzigen Fall dem durch die vorliegende Erfindung erreichten Zweck einer auf einfache Weise erzielbaren wirkungsvollen Maskierung des bitter-brennenden Geschmacks von Propionsäure-Derivaten, also insbedondere von Dexibuprofen.

Im Sinne einer angestrebten möglichst wirksamen Unterdrückung bzw. Maskierung des extrem unangenehmen Geschmacks der Propionsäure-Derivate bzw. Profene hat es sich als besonders vorteilhaft erwiesen, in der neuen pharmazeutischen Zubereitung einen beim Suspendieren derselben in Wasser für die Bereitung einer Trink-Zubereitung den pH-Wert derselben auf einen Wert von unter 7 senkenden Hilfsstoff einzusetzen, wozu bezüglich näherer Details auf den Anspruch *3* hingewiesen sei.

Für den Patienten besonders gut annehmbar sind - wie sich gezeigt hat - TrinkZubereitungen auf Basis der neuen pharmazeutischen Zubereitung gemäß Anspruch *4*, gemäß welchem zur Geschmacks-Verbesserung der Einsatz mindestens eines Süßungsmittels und/oder Aromastoffes vorgesehen ist.

Als Süßungsmittel eignen sich besonders Zucker und Zuckeralkohole, wie Saccharose, Glucose, Fructose, Maltose bzw. Sorbit, Xylit, Mannit. Des Weiteren können künstliche Süßstoffe wie Saccharin, Acesulfam, Cyclamat, Aspartam sowie deren Salze zugesetzt sein.

Aufgrund des sauren pH-Wertes der aus der neuen pharmazeutischen Zubereitung herstellbaren trinkfertigen Suspension empfehlen sich zur Aromatisierung insbesondere, aber nicht ausschließlich, Fruchtaromen wie Orangen-, Zitronen-, Johannisbeer-, Kirschen-, Himbeer-, Apfel- und Bimen-Aroma.

Diese Aufzählungen sind bloß beispielhaft und nicht vollständig.

Weiters kann - wie dem Anspruch *5* zu entnehmen - jeweils in Abhängigkeit von der gewählten Arzneiform der Einsatz weiterer Hilfsstoffe günstig oder notwendig sein, die üblicher Weise bei der Herstellung von Granulaten und Tabletten eingesetzt werden, wie z.B. Bindemittel, Füllmittel, Schmiermittel, Fließregulierungsmittel u.dgl.

Als weitere Bestandteile der neuen pharmazeutischen Zubereitung können, siehe dazu den Anspruch *6*, oberflächenaktive Hilfsstoffe zugesetzt werden, die eine rasche Benetzung und homogene Dispergierung des an sich wie bekannt, schlecht wasserlöslichen Wirkstoffes ermöglichen. Für diesen Zweck eignen sich - keineswegs ausschließlich - Natriumlaurylsulfat, Polyethylenglykolfettalkoholether oder Polyethylenglykolfettsäueester.

Wie im Anspruch *7* geoffenbart, können weiters auch hydrophile Kolloide zugesetzt werden. Dazu zählen insbesondere Hydroxypropylmethylcellulose, Hydroxypropylcellulose, Methylcellulose, Hydroxyethylcellulose, Natriumcarboxymethylcellulose, Polyvinylpyrrolidon u.dgl. Es empfiehlt sich jedoch die Verwendung niedrigviskoser Typen und/oder der Einsatz geringer Mengen dieser Zusätze, so dass die Viskosität der trinkfertigen Zubereitung im Vergleich zu jener von Wasser nicht wesentlich erhöht wird.

Bevorzugt kann es weiters sein, wenn, wie gemäß Anspruch *8* vorgesehen, die neue Arzneimittel-Zubereitung als Brauseformulierung vorliegt.

Dafür eignet sich der Einsatz von Carbonaten, insbesondere von Natriumhydrogencarbonat oder Natriumcarbonat, alleine oder in Kombination miteinander. Die Menge an Carbonat hängt von der Gesamtmenge Säure in der Zubereitung ab und wird vorteilhaft derart gewählt, dass der pH-Wert der trinkfertigen Zubereitung unter 7 bleibt.

Einen weiteren wesentlichen Gegenstand der Erfindung stellt das neue Verfahren zur Herstellung der pharmazeutischen Zubereitung in ihrer Grundform und in den bevorzugten Ausbildungsformen dar, wie im Anspruch *9* geoffenbart.

Besonders bevorzugt ist eine Ausführungsform des Herstellungs-Verfahrens gemäß Anspruch *10*, wobei dort der Einsatz von Wirkstoff-Partikeln mit engeren Korngrößen-Fraktionsbereichen vorgesehen ist.

Einen weiteren wesentlichen Gegenstand der Erfindung stellt die unter Einsatz der neuen Arzneimittel-Zubereitungen herstellbaren Trinkzubereitung gemäß Anspruch *11* dar, so wie deren in den Ansprüchen *12* bis *14* beschriebene Ausführungsformen.

Weiters stellt die Herstellung der neuen Trink-Zubereitung gemäß Anspruch *15* einen Gegenstand der Erfindung dar und schließlich auch die für die vorliegende Erfindung wesentliche Verwendung von Popionsäure-Derivat-Partikeln genau definierter Korngrößen-Bereiche gemäß Anspruch *16*.

Die Herstellung der festen Arzneiform für die Bereitung einer Trinksuspension kann durch einfache pharmazeutische Verfahrens-Vorgänge, wie Sieben, Mischen, Granulieren und Tablettieren u.dgl. erfolgen. Die Einzeldosierung von Pulvern und Granulaten kann durch das Abfüllen entsprechender Mengen in Sachets erfolgen. Die Menge Wirksubstanz einer Einzeldosis kann durch das Füllgewicht bzw. das Tablettengewicht oder durch den relativen Anteli an Wirkstoff, wie Dexibuprofen, in der fertigen Mischung gesteuert werden.

Im Fall von Dexibuprofen beträgt die pharmakologisch wirksame Menge an Wirkstoff pro Einzeldosis bevorzugt zwischen 100 und 400 mg, zumindest jedoch 50 mg.

Anhand der Beispiele wird die Erfindung näher erläutert:

### Beispiel 1:

Herstellung einer Charge Dexibuprofen-Trinkpulver mit 67,8 kg:

| Bestandteil | Menge |
|---|---|
| Dexibuprofen 125 - 500 µm | 6,000 kg |
| Zitronensäure | 18,000 kg |
| Saccharin-Natrium | 1,500 kg |
| Saccharose | 36,000 kg |
| Natriumlaurylsulfat | 0,030 kg |
| Siliziumdioxid | 0,300 kg |
| Orangen-Aroma | 6,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 2261 mg | 200 mg |
| 3392 mg | 300 mg |
| 4522 mg | 400 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Trinksuspension schmeckt angenehm und hinterlässt keine Geschmacksirritationen. Beispiel 2:

Herstellung einer Charge Dexibuprofen-Trinkpulver mit 42,7 kg:

| Bestandteil | Menge |
|---|---|
| Dexibuprofen 125 - 500 µm | 2,000 kg |
| Zitronensäure | 12,000 kg |
| Saccharin-Natrium | 0,500 kg |
| Saccharose | 24,000 kg |
| Natriumlaurylsulfat | 0,010 kg |
| Siliziumdioxid | 0,200 kg |
| Zitronen-Aroma | 4,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 2136 mg | 100 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Trinksuspension schmeckt angenehm und hinterlässt keine Geschmacksirritationen.

### Beispiel 3:

Herstellung einer Charge Dexibuprofen-Brausepulver mit 65,9 kg:

| Bestandteil | Menge |
|---|---|
| Dexibuprofen 200 - 400 µm | 6,000 kg |
| Natriumcarbonat | 6,000 kg |
| Zitronensäure | 21,000 kg |
| Saccharin-Natrium | 1,500 kg |
| Saccharose | 27,000 kg |
| Natriumlaurylsulfat | 0,030 kg |
| Orangen-Aroma | 4,350 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 1098 mg | 100 mg |
| 2197mg | 200 mg |
| 3295 mg | 300 mg |
| 4393 mg | 400 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt bei ca. 4. Die Brausezubereitung schmeckt angenehm und hinterlässt keine Geschmacksirritationen.

### Beispiel 4:

Herstellung einer Charge Dexibuprofen-Brausepulver mit 67,4 kg:

| Bestandteil | Menge |
|---|---|
| Dexibuprofen 200 - 400 µm | 6,000 kg |
| Natriumcarbonat | 6,000 kg |
| Zitronensäure | 21,000kg |
| Saccharin-Natrium | 1,500 kg |
| Saccharose | 27,000 kg |
| Natriumlaurylsulfat | 0,030 kg |
| Hydroxypropylmethylcellulose | 1,500 kg |
| Orangen-Aroma | 4,350 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 1123 mg | 100 mg |
| 2246 mg | 200 mg |
| 3369 mg | 300 mg |
| 4492 mg | 400 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt bei ca. 4. Die Brausezubereitung schmeckt angenehm und hinterlässt keine Geschmacksirritationen.

### Beispiel 5:

Herstellung einer Charge Dexibuprofen-Trinkpulver, zuckerfrei mit 45,2 kg:

| Bestandteil | Mengen |
|---|---|
| Dexibuprofen 125 - 500 µm | 4,000 kg |
| Zitronensäure | 12,000 kg |
| Saccharin-Natrium | 1,000 kg |
| Xylit | 24,000 kg |
| Natriumlaurylsulfat | 0,020 kg |
| Aerosil 200 | 0,200 kg |
| Orangen-Aroma | 4,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 4522 mg | 400 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Zubereitung schmeckt angenehm und hinterlässt keine Geschmacksirritationen.

### Beispiel 6:

Herstellung einer Charge Dexibuprofen-Trinkpulver, zuckerfrei mit 45,2 kg:

| Bestandteil | Menge |
|---|---|
| Dexibuprofen 125 - 500 µm | 4,000 kg |
| Zitronensäure | 12,000 kg |
| Saccharin-Natrium | 1,000 kg |
| Mannit | 24,000 kg |
| Natriumlaurylsulfat | 0,020 kg |
| Aerosil 200 | 0,200 kg |
| Johannisbeer-Aroma | 4,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 3392 mg | 300 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Zubereitung schmeckt angenehm und hinterlässt keine Geschmacksirritationen.

### Beispiel 7:

Herstellung einer Charge Ibuprofen-Trinkpulver, zuckerfrei mit 45,2 kg:

| Bestandteil | Menge |
|---|---|
| Ibuprofen 125 - 500 µm | 4,000 kg |
| Zitronensäure | 12,000 kg |
| Saccharin-Natrium | 1,000 kg |
| Sorbit | 24,000 kg |
| Natriumlaurylsulfat | 0,020 kg |
| Aerosil 200 | 0,200 kq |
| Himbeer-Aroma | 4,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 2261 mg | 200 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Zubereitung schmeckt angenehm und hinterlässt keine Geschmacksirritationen.

### Beispiel 8:

Herstellung einer Charge Dexibuprofen-Tabletten mit 25 kg:

| Bestandteil | Menge |
|---|---|
| Dexibuprofen 200 - 500 µm | 2,000 kg |
| Zitronensäure | 6,000 kg |
| Saccharin-Natrium | 0,500 kg |
| Saccharose | 9,000 kg |
| Natriumlaurylsulfat | 0,010 kg |
| Aerosil 200 | 0,100 kg |
| Orangen-Aroma | 2,000 kg |
| Polyvinylpyrrolidon, quervernetzt | 1,000 kg |
| Mikrokristalline Cellulose | 4,090 kg |
| Magnesiumstearat | 0,300 kg |

Die Bestandteile werden in einem Freifallmischer bis zur homogenen Durchmischung gemischt und auf einer Tablettenmaschine verpresst.

Das Tablettensollgewicht entspricht 2500 mg entsprechend 200 mg Dexibuprofen pro Tablette. Die Tablette zerfällt in einem Glas Leitungswasser binnen 1 Minute und ergibt eine wohlschmeckende Trinksuspension.

### Beispiel 9:

Herstellung einer Charge Flurbiprofen-Trinkpulver mit 62,8 kg:

| Bestandteil | Menge |
|---|---|
| Flurbiprofen 125 - 500 µm | 1,000 kg |
| Zitronensäure | 18,000 kg |
| Saccharin-Natrium | 1,500 kg |
| Saccharose | 36,000 kg |
| Natriumlaurylsulfat | 0,030 kg |
| Siliziumdioxid | 0,300 kg |
| Orangen-Aroma | 6,000 kg |

Die Bestandteile werden in einem Freifallmischer bis zur homogenen Durchmischung gemischt.

Die Abteilung in Einzeldosen erfolgt durch Abfüllen in Sachets. Entsprechend der Füllmenge kann die Wirkstoffmenge gesteuert werden:

| Füllmenge pro Sachet | Menge Dexibuprofen pro Sachet |
|---|---|
| 3141,5 mg | 50 mg |
| 6283 mg | 100 mg |

Der Inhalt eines Sachets wird in einem Glas Wasser (ca. 150 bis 250 ml) dispergiert. Nach kurzem Umrühren ist die Zubereitung trinkfertig. Der pH-Wert liegt zwischen 2 und 3. Die Trinksuspension schmeckt angenehm und hinterlässt keine Geschmacksirritationen.

Nachweis der Geschmacksmaskierung:

Es wurde ein Geschmackstest mit Dexibuprofen-Pulver zum Herstellen einer Trinksuspension durchgeführt. Ein Sachet enthielt 3369 mg der Mischung aus Beispiel 4 entsprechend 300 mg Dexibuprofen. Das Pulver wurde unmittelbar vor der Anwendung in 125 ml Leitungswasser dispergiert und verkostet. An dem Versuch nahmen 65 Freiwillige teil. Tabelle 1 zeigt eine Übersicht zur Versuchsgruppe, Tabelle 2 die Ergebnisse des Geschmackstestes. Es wurde sowohl der Erst-Eindruck als auch der Nachgeschmack beurteilt.

Die Ergebnisse belegen die hohe Geschmacksakzeptanz der erfindungsgemäßen Zubereitung.

**Tab. 1: Versuchsgruppe Geschmackstest Dexibuprofen-Brausepulver 300 mg**

| Durchschnittsalter | 37 Jahre | |
|---|---|---|
| Gesamtzahl Probanden | 65 | |
| Männlich | 35 | 53,8 % |
| Weiblich | 30 | 46,2 % |

**Tab. 2: Ergebnisse des Geschmackstestes getrennt nach Erst-Eindruck und Nachgeschmack**

| Geschmacksbeurteilung | Erst-Eindruck | Nachgeschmack |
|---|---|---|
| ausgezeichnet | 13,8 % | 12,3 % |
| gut | 52,3 % | 58,5 % |
| akzeptabel | 21,5 % | 16,9 % |
| trinkbar | 9,2 % | 10,8 % |
| nicht genießbar | 3,1 % | 1,5 % |

## Patentansprüche

1. Pharmazeutische Zubereitung in fester Pulver-, Granulat- oder Tablettenform, welche mindestens ein bitteren bzw. brennenden Geschmack aufweisendes Propionsäure-Derivat, vorzugsweise Dexibuprofen oder racemisches Ibuprofen, als Wirkstoff sowie weiters pharmazeutische Hilfs- und Zusatzstoffe enthält, für die Bereitung einer Trink-Zubereitung durch Dispergierung in Wasser, **dadurch gekennzeichnet, dass** der genannte bittere bzw. brennende Geschmack der mit der Feststoff-Zubereitung hergestellten Trink-Zubereitung unterdrückt, maskiert bzw. vermieden ist, indem das in der Feststoff-Zubereitung jeweils enthaltene Propionsäure-Derivat in Form von - von Partikeln aller anderen Korngrößen-Fraktionen im Wesentlichen befreiten, zumindest zu 95 %, der Korngrößen-Fraktion von 100 bis 1000 µm angehörenden - Partikeln vorliegt,
und die
Propionsäure-Derivat-Partikel keinen Überzugsfilm, *insbesondere* keinen, deren Kontakt bzw. Benetzung mit Wasser hemmenden Überzugsfilm, aufweisen.

2. Pharmazeutische Zubereitung nach Anspruch 1, **dadurch gekennzeichnet, dass** die in ihr enthaltenen Propionsäure-Derivat-Partikel zumindest zu 95 % der Korngrößen-Fraktion von 125 bis 500 µm, und insbesondere von 200 bis 400 µm, angehören.

3. Pharmazeutische Zubereitung nach Anspruch 1, **dadurch gekennzeichnet, dass** in derselben zumindest ein beim Kontakt mit Wasser ein Lösen des Propionsäure-Derivats hemmender, den pH-Wert der mit der Zubereitung bereiteten Trink-Zubereitung auf einen Wert von unter 7, bevorzugt auf einen Wert im Bereich von 2 bis 4, senkender bzw. einstellender Hilfsstoff, wie insbesondere mindestens eine feste organische Säure, vorzugsweise Zitronensäure, Weinsäure, Äpfelsäure, Gluconsäure, Ascorbinsäure, Fumarsäure und/oder Bernsteinsäure, und/oder aber mindestens eine sauer reagierende (Puffer-)Substanz, wie insbesondere Natriumdihydrogenphosphat, enthalten ist.

4. Pharmazeutische Zubereitung nach einem der Ansprüche 1 bis 3, **dadurch gekennzeichnet, dass** sie zur Geschmacksverbesserung mindestens ein Süßungsmittel und/oder mindestens einen Aromastoff enthält.

5. Pharmazeutische Zubereitung nach einem der Ansprüche 1 bis 4, **dadurch gekennzeichnet, dass** sie für die Arzneiformung und Herstellung von Pulvern, Granulaten und Tabletten übliche Hilfsstoffe, wie insbesondere Bindemittel, Füll-, Schmier- und/oder Fließregulierungsmittel, enthält.

6. Pharmazeutische Zubereitung nach einem der Ansprüche 1 bis 5, **dadurch gekennzeichnet, dass** sie zumindest einen benetzungsfördernden Zusatzstoff, insbesondere aus der Gruppe der pharmazeutisch akzeptablen Tenside und Öl-in-Wasser-Emulgatoren, enthält.

7. Pharmazeutische Zubereitung nach einem der Ansprüche 1 bis 6, **dadurch gekennzeichnet, dass** sie mindestens ein hydrophiles Kolloid, bevorzugt mit niedriger Molekularmasse, enthält.

8. Pharmazeutische Zubereitung nach einem der Ansprüche 1 bis 7, **dadurch gekennzeichnet, dass** in derselben - für die Bereitung von Brause-TrinkZubereitungen - mindestens ein mit der in der Zubereitung enthaltenen Säure oder sauer reagierenden Substanz Kohlendioxid freisetzendes Carbonat enthalten ist.

9. Verfahren zur Herstellung einer pharmazeutischen Zubereitung gemäß einem der Ansprüche 1 bis 8, wobei Partikel des Propionsäure-Derivats mit pharmazeutischen Zusatz- und Hilfsstoffen gemischt und in eine für eine rasche Dispergierung in Wasser vorgesehene, feste Form, insbesondere Pulver-, Granulat- oder Tabletten-Form gebracht werden, **dadurch gekennzeichnet, dass** aus dem jeweiligen, in Partikel-Form vorliegenden Wirkstoff durch Korngrößenklassierung, Sieben und/oder Sichten der Feinkorn-Anteil mit Korngrößen von unter 100 µm, sowie ein eventueller Grob-Anteil von über 1000 µm bis zum Vorliegen von höchstens 5 % außerhalb der genannten Partikelgrößen-Bereichsgrenzen liegender Wirkstoff-Partikeln von höchstens 5 % außerhalb der Partikelgrößen-Bereichsgrenzen liegenden Partikeln der Korngrößen-Fraktion von 100 bis 1000 µm entfernt wird und die Proprionsäure-Derivat-Partikel durch schonendes Mischen unter Vermeidung von Frakturen, bevorzugt durch Freifall-Mischen, mit jeweils vorgesehenen pharmazeutischen Hilfs- und Zusatzstoffen homogen vermengt werden.

10. Verfahren nach Anspruch 9, **dadurch gekennzeichnet, dass** zu zumindest 95 % der Korngrößen-Fraktion von 125 bis 500 µm, vorzugsweise der Fraktion von 200 bis 400 µm, angehörende und von Partikeln mit Größen außerhalb dieser Bereichsgrenzen befreite Propionsäure-Derivat-Partikel eingesetzt werden.

11. Trinkbare pharmazeutische Zubereitung mit organoleptisch akzeptablem bzw. gutem, Geschmack, enthaltend mindestens ein Propionsäure-Derivat bzw. Profen, vorzugsweise Dexibuprofen oder racemisches Ibuprofen, als Wirkstoff, **dadurch gekennzeichnet, dass** sie unmittelbar vor der Einnahme bzw. Verabreichung durch Dispergieren der in fester Form vorliegenden pharmazeutischen Zubereitung gemäß einem der Ansprüche 1 bis 8 bzw. der gemäß Anspruch 9 oder 10 hergestellten pharmazeutischen Zubereitung in Wasser bereitet ist, wobei sich das jeweilige Propionsäure-Derivat nicht löst, und wobei die Korngröße der in der Trink-Zubereitung suspendierten Wirkstoff-Partikel zumindest zu 95 % zwischen 100 µm und 1000 µm, liegt.

12. Trink-Zubereitung nach Anspruch 11, **dadurch gekennzeichnet, dass** die Korngröße der suspendierten Proprionsäure-Derivat-Partikel jeweils zumindest zu 95 % zwischen 125 µm und 500 µm, bevorzugt zwischen 200 µm und 400 µm, liegt.

13. Trink-Zubereitung nach Anspruch 11 oder 12, **dadurch gekennzeichnet, dass** sie eine niedrige Viskosität, von bis maximal 75 mPa·s, vorzugsweise im Bereich von 1 bis 15 mPa·s, aufweist.

14. Trink-Zubereitung nach einem der Ansprüche 11 bis 13, **dadurch gekennzeichnet, dass** ihr pH-Wert unter 7, und bevorzugt zwischen 2 und 4, liegt.

15. Verfahren zur Herstellung einer trinkbaren pharmazeutischen Zubereitung bzw. Trink-Zubereitung nach einem der Ansprüche 11 bis 14, **dadurch gekennzeichnet, dass** eine in fester Form vorliegende pharmazeutische Zubereitung gemäß einem der Ansprüche 1 bis 8, bzw. eine gemäß Anspruch 9 oder 10 hergestellte derartige Zubereitung unmittelbar vor Einnahme bzw. Verabreichung durch rasches Dispergieren und Ein-und Umrühren in 100 bis 250 ml Wasser pro 200, 300 bzw. 400g Wirkstoff Dexibuprofen in der trinkbaren Zubereitung, in eine trinkfertige Suspension übergeführt wird.

16. Verwendung von Partikeln mindestens eines Wirkstoffes aus der Gruppe der Propionsäure-Derivate bzw. Profene, vorzugsweise Dexibuprofen oder racemisches Ibuprofen, als Wirkstoff mit zu zumindest 95 % innerhalb der Korngrößen-Fraktion von 100 bis 1000 µm liegender Partikelgröße für die Herstellung von pharmazeutischen Zubereitungen in fester Form gemäß einem der Ansprüche 1 bis 10, welche in TrinkZubereitungen zu überführen sind, für die Unterdrückung, Maskierung bzw. Vermeidung des bei Überführung der Propionsäure-Derivate bzw. Profene enthaltenden, in fester Form vorliegenden pharmazeutischen Zubereitungen in TrinkZubereitungen gemäß einem der Ansprüche 11 bis 15 auftretenden inakzeptabel bitteren bzw. brennenden Geschmackes dieser Wirkstoffe.

## Claims

1. A pharmaceutical preparation in a solid powder, granulate, or tablet form, which contains at least one propionic acid derivative having a bitter and/or burning taste, preferably dexibuprofen or racemic ibuprofen, as an active agent as well as pharmaceutical additives and adjuvants, for preparing a drink preparation by dispersion in water, **characterized in that** said bitter and/or burning taste of the drink preparation is suppressed, masked or prevented by the propionic acid derivative contained in each solid preparation, which is present in the form of particles of the grain size fraction from 100 to 1000 µm, at least 95 % of which are substantially free of particles of any other grain size fractions, and **in that** said propionic acid derivative particles do not have a coating film, in particular none that inhibits their contact and/or wetting with water.

2. The pharmaceutical preparation according to claim 1, **characterized in that** at least 95 % of the propionic acid derivative particles contained therein are parts of the grain size fraction from 125 to 500 µm, particularly from 200 to 400 µm.

3. The pharmaceutical preparation according to claim 1, **characterized in that** it contains at least one adjuvant such as, in particular, at least one solid organic acid, preferably citric acid, tartaric acid, malic acid, gluconic acid, ascorbic acid, fumaric acid and/or succinic acid, and/or at least one acidic (buffer) substance such as, in particular, sodium dihydrogen phosphate, which adjuvant inhibits dissolution of said propionic acid derivative and reduces and/or adjusts the pH value of the drink preparation prepared using said preparation to a value below 7, preferably to a value ranging from 2 to 4.

4. The pharmaceutical preparation according to any of claims 1 to 3, **characterized in that** it contains at least one sweetening agent and/or at least one flavoring agent for improving taste.

5. The pharmaceutical preparation according to any of claims 1 to 4, **characterized in that** it contains adjuvants commonly used for forming drugs and preparing powders, granulates, and tablets, such as, in particular, binding agents, filling agents, lubricants, and/or flow-regulating agents.

6. The pharmaceutical preparation according to any of claims 1 to 5, **characterized in that** it contains at least one additive which supports wetting, in particular one selected from the group consisting of pharmaceutically acceptable surfactants and oil-in-water emulsifying agents.

7. The pharmaceutical preparation according to any of claims 1 to 6, **characterized in that** it contains at least one hydrophilic colloid, preferably having a low molecular mass.

8. The pharmaceutical preparation according to any of claims 1 to 7, **characterized in that** it contains at least one carbonate releasing carbon dioxide when reacting with the acid or acidic substance contained in the preparation, for preparing effervescent drink preparations.

9. A method for preparing a pharmaceutical preparation according to any of claims 1 to 8, wherein particles of said propionic acid derivative are mixed with pharmaceutical additives and adjuvants and brought into a solid form intended for quick dispersion in water, in particular into powder, granulate, or tablet form, **characterized in that** the fine-grain content with grain sizes below 100 µm, as well as any coarse-grain content with sizes of more than 1000 µm is removed from each active agent present in particulate form by grain size sorting, sieving and/or screening until the proportion of active agent particles beyond the particle size range of the grain size fraction from 100 to 1000 µm amounts to max. 5 %, and the propionic acid derivative particles are mixed homogeneously with respective pharmaceutical additives and adjuvants by gentle mixing while preventing fractures, preferably by free-fall mixing.

10. The method according to claim 9, **characterized in that** propionic acid derivative particles are used, at least 95 % of which belong to the grain-size fraction from 125 to 500 µm, preferably to the fraction from 200 to 400 µm, and are free from particles having sizes beyond these range limitations.

11. A drinkable pharmaceutical preparation having organoleptically acceptable and/or good taste, containing at least one propionic acid derivative and/or profen, preferably dexibuprofen or racemic ibuprofen, as an active agent, **characterized in that** it is prepared immediately prior to intake and/or administration by dispersing the pharmaceutical preparation present in a solid form according to any of claims 1 to 8 or the pharmaceutical preparation prepared according to any of claims 9 and 10 in water, wherein the respective propionic acid derivative does not dissolve, and wherein the grain size of at least 95 % of the active agent particles suspended in the drink preparation is between 100 µm and 1000 µm.

12. The drink preparation according to claim 11, **characterized in that** the grain size of at least 95 % of the propionic acid derivative particles suspended in the drink preparation is between 125 µm and 500 µm, preferably between 200 µm and 400 µm.

13. The drink preparation according to claim 11 or 12, **characterized in that** it has a low viscosity of up to 75 mPa·s, preferably in the range of 1 to 15 mPa·s.

14. The drink preparation according to any of claims 11 to 13, **characterized in that** it has a pH value below 7, and preferably between 2 and 4.

15. A method for preparing a drinkable pharmaceutical preparation and/or drink preparation according to any of claims 11 to 14, **characterized in that** a pharmaceutical preparation present in a solid form according to any of claims 1 to 8 and/or such a preparation prepared according to claim 9 or 10 is converted to a ready-to-drink suspension immediately prior to intake and/or administration by quickly dispersing and stirring said preparation in 100 to 250 ml of water per 200, 300 or 400 g, respectively, of the active ingredient dexibuprofen contained in the drinkable preparation, followed by agitating.

16. A use of particles of at least one active agent selected from the group consisting of propionic acid derivative and/or profens, preferably dexibuprofen or racemic ibuprofen, as an active agent, at least 95 % of which have a grain size within the grain size fraction from 100 to 1000 µm, for preparing pharmaceutical preparations in a solid form according to any of claims 1 to 10, which are to be converted into drink preparations, for suppressing, masking and/or preventing said unacceptable bitter and/or burning taste of said active agents occurring upon converting the pharmaceutical preparations present in a solid form which contain propionic acid derivatives and/or profen into drink preparations according to any of claims 11 to 15.

## Revendications

1. Préparation pharmaceutique en forme d'une poudre, d'un granulat ou d'un comprimé, ladite préparation contenant, comme principe actif, au moins un dérivé d'acide propionique au goût amer ou brûlant, de préférence du dexibuprofène ou de l'ibuprofène racémique, ainsi que d'autres additifs et adjuvants pharmaceutiques, pour la préparation d'une préparation potable par sa dispersion dans l'eaux, **caractérisée en ce que** ledit goût amer ou brûlant de ladite préparation potable préparée à partir de ladite préparation solide est supprimé, masqué ou évité par le fait que le dérivé respectif d'acide propionique contenu dans ladite préparation solide est présent en forme de particules, de particules dont au moins 95 % appartiennent à la fraction des dimensions de particules de 100 à 1000 µm et qui sont sensiblement exemptes de particules de toutes autres fractions de dimensions, et **en ce que** lesdites particules dudit dérivé d'acide propionique n'ont pas de film d'enrobage, notamment aucun film qui inhibe leur contact avec ou leur mouillage par de l'eau.

2. Préparation pharmaceutique selon la revendication 1, **caractérisée en ce qu'**au moins 95 % des particules de dérivé d'acide propionique qu'elle contient appartiennent à la fraction de dimensions de 125 à 500 µm, notamment de 200 à 400 µm.

3. Préparation pharmaceutique selon la revendication 1, **caractérisée en ce qu'**elle contient au moins un adjuvant, notamment au moins un acide organique solide, de préférence d'acide citrique, d'acide tartrique, d'acide malique, d'acide gluconique, d'acide ascorbique, d'acide fumarique et/ou d'acide succinique, et/ou au moins une substance (tampon) acide, notamment d'hydrogénophosphate de sodium, pour inhiber la dissolution dudit dérivé d'acide propionique quand ceci est contacté avec de l'eau, pour baisser ou ajuster le pH de la préparation potable préparée en utilisant ladite préparation pour qu'il soit inférieur à 7, de préférence entre 2 à 4.

4. Préparation pharmaceutique selon l'une des revendications 1 à 3, **caractérisée en ce qu'**elle contient au moins un édulcorant et/ou au moins une flaveur pour améliorer son goût.

5. Préparation pharmaceutique selon l'une des revendications 1 à 4, **caractérisée en ce qu'**elle contient des adjuvants habituellement utilisés pour la préparation de médicaments et de poudres, de granulats et de comprimés, notamment des matières liantes, des matières de remplissage, des matières lubrifiantes et/ou de matières de réglage de fluidité.

6. Préparation pharmaceutique selon l'une des revendications 1 à 5, **caractérisée en ce qu'**elle contient au moins un adjuvant promouvant la mouillage, notamment choisi parmi le groupe comprenant les surfactants pharmaceutiquement acceptables et les émulsifiants pour la préparation d'émulsions huile dans l'eau.

7. Préparation pharmaceutique selon l'une des revendications 1 à 7, **caractérisée en ce qu'**elle contient au moins un colloïde hydrophile, de préférence à poids moléculaire bas.

8. Préparation pharmaceutique selon l'une des revendications 1 à 7, **caractérisée en ce qu'**elle contient au moins un carbonate qui libère du gaz carbonique quand il réagit avec ledit acide ou ladite substance acide contenu/e dans ladite préparation, pour préparer des préparations potables effervescentes.

9. Procédé pour préparer une préparation pharmaceutique selon l'une des revendications 1 à 8, des particules de dérivé d'acide propionique étant mélangées avec des additifs et adjuvants pharmaceutiques et mises dans une forme solide, notamment une forme de poudre, de granulat ou de comprimé, **caractérisé en ce que**, par triage, tamisage et/ou classement, la fraction des grains fins avec des dimensions inférieures à 100 µm ainsi qu'une fraction éventuelle des gros grains de plus de 1000 µm sont éliminées du principe actif respectif en forme de particules jusqu'à ce que le pourcentage des particules de principe actif avec des dimensions qui sont en dehors des limites de la fraction avec des dimensions des particules de 100 à 1000 µm atteigne 5 % au maximum, et **en ce que** les particules de dérivé d'acide propionique sont mélangées avec ménagement, en évitant des fractures, et de manière homogène avec les additifs et adjuvants respectivement prévus, de préférence par un procédé de mélange par chute libre.

10. Procédé selon la revendication 9, **caractérisé en ce que** des particules de dérivé d'acide propionique dont au moins 95 % appartiennent à la fraction avec des dimensions des particules de 125 à 500 µm, de préférence de la fraction de 200 à 400 µm, et qui sont exemptes de particules avec des dimensions en dehors ces limites sont utilisées.

11. Préparation pharmaceutique potable au goût organoleptiquement acceptable ou au bon goût, contenant, comme principe actif, au moins un dérivé d'acide propionique ou au moins un profène, de préférence du dexibuprofène ou de l'ibuprofène racémique, **caractérisée en ce que** ladite préparation est préparée immédiatement avant être prise ou administrée par la dispersion d'une préparation pharmaceutique solide selon l'une des revendications 1 à 8 ou selon la revendication 9 ou 10 dans l'eau, le dérivé respectif d'acide propionique n'étant pas dissous et les dimensions d'au moins 95 % des particules de principe actif suspendues dans la préparation potable étant entre 100 µm et 1000 µm.

12. Préparation potable selon la revendication 11, **caractérisée en ce que** les dimensions d'au moins 95 % des particules de dérivé d'acide propionique suspendues sont entre 125 µm et 500 µm, de préférence entre 200 µm et 400 µm.

13. Préparation potable selon la revendication 11 ou 12, **caractérisée en ce qu'**elle a une viscosité basse de 75 mPa·s, au maximum, de préférence de 1 à 15 mPa·s.

14. Préparation potable selon l'une des revendications 11 à 13, **caractérisée en ce que** son pH est inférieur à 7, de préférence entre 2 et 4.

15. Procédé pour la préparation d'une préparation pharmaceutique ou d'une préparation pharmaceutique potable selon l'une des revendications 11 à 14, **caractérisé en ce qu'**une préparation pharmaceutique solide selon l'une des revendications 1 à 8 ou une telle préparation préparée selon l'une des revendications 9 à 10 est convertie en suspension prête-à-boire immédiatement avant être prise ou administrée, par être rapidement dispersée, délayée et agitée dans 100 à 250 ml d'eau par 200, 300 ou 400 g de principe actif dexibuprofène.

16. Utilisation de particules d'au moins un principe actif choisi parmi le groupe des dérivés d'acide propionique et des profènes, de préférence de dexibuproféne ou d'ibuprofène racémique, dont au moins 95 % ont des dimensions appartenant à la fraction de 100 à 1000 µm, pour la préparation de préparations pharmaceutiques solides selon l'une des revendications 1 à 10 qui sont à convertir en préparations potables, pour supprimer, masquer ou éviter un goût inacceptablement amer ou brûlant desdits principes actifs qui se produit quand lesdites préparations pharmaceutiques solides, comprenant des dérivés d'acide propionique ou des profènes, sont converties en préparations potables selon l'une des revendications 11 à 15.
